# EUROPEAN PATENT SPECIFICATION

(11) **EP 0 322 677 B1**
(45) Date of publication and mention of the grant of the patent: **10.08.1994**
(21) Application number: 88121070.2
(22) Date of filing: 16.12.1988
(51) Int. Cl.: C07H 21/04

(54) **Carrier for DNA-hybridization**
Träger für DNS-Hybridisierung
Support pour hybridation d'adn

(30) Priority: 25.12.1987 JP 329402/87
(43) Date of publication of application: 05.07.1989
(73) Proprietor: F. HOFFMANN-LA ROCHE AG, 4002 Basel (CH)
(72) Inventor: Furuichi, Yasuhiro, Kamakura-shi Kanagawa-ken (JP); Hikata, Mikio, Yokohama-shi Kanagawa-ken (JP); Kuribayashi, Keiko, Yokohama-shi Kanagawa-ken (JP)
(74) Representative: Notegen, Eric-André

(56) References cited:
- EP-A- 0 101 985
- EP-A- 0 180 945
- JOURNAL OF THE AMERICAN CHEMICAL SOCIETY, vol. 86, no. 22, 20th November 1964, pages 4982-4985; P.T. GILHAM: "The synthesis of polynucleotide-celluloses and their use in the fractionation of polynucleotides"

## Description

This invention relates to a hybridization carrier which is useful for the detection, the isolation, the purification of nucleic acid, especially of messenger RNA(mRNA) containing polyadenylic acid sequence, and a process for preparing the same.

A solid support with a nucleic acid immobilized on its surface is known as a useful material for the techniques of detecting, isolating and purifying nucleic acids containing the desired specific base sequence. This technique is a method developed by scientists in the field of recombinant DNA technology, the application of which is based on the nature that a nucleic acid of DNA or RNA denatured to the single-stranded form can hybridize with other single-stranded nucleic acids containing substantially complementary base sequences through the hydrogen bonding between the bases under suitable conditions.

As a method of immobilizing a single-stranded nucleic acid on the surface of solid support, a method was developed which comprised bringing a solid support composed of such materials having high affinities for nucleic acids as nitrocellulose or nylon membranes into contact with a nucleic acid, trapping the nucleic acid on the support and then giving the baking or UV irradiation to the resulting support so as to strengthen the immobilization. This method, however, has a problem in terms of that the nucleic acids that lack the desired specific base sequence in a sample or the nucleic acid detection probe is also liable to be trapped on the surface of the support because of the high affinity of solid support to nucleic acids. Thus, in order to prevent the nucleic acid from the unspecific trap, this method indispensably requires very tedius operations of coating the surface of the support with a polymeric substance foreign to the desired specific base sequence, besides, repeated washing of the support after a hybrid is formed.

In order to solve the disadvantages described above, various nucleic acid immobilization supports and their manufacturing processes are being proposed. Among these, a support that immobilizes a nucleic acid through carbon chain having 4 to 20 carbon atoms (part of the carbon atoms may be substituted with a heteroatom such as O, N, S or the like and this chain is referred to as "arm") between a nucleotide sequence part constituting the nucleic acid and a support. Said nucleic acid immobilization support is manufactured by previously activating a given part of a base in a nucleotide as will be an immobilization site and then reacting said activated part with a reactive residue provided for the support as an "arm" to form a covalent bond (Japanese Patent Application Kokai No. 130305/1986). Furthermore, there is known a method of ligating nucleic acid to a support, on which a nucleotide or an oligonucleotide is to be immobilized, by utilizing the enzymatic reaction (Japanese Patent Application Kokai No. 246201/1986).

However, in the method described in the official gazette of Japanese Patent Application Kokai No. 130305/1986, it is required to protect other reactive residues in a nucleic acid by protective residues at the time of activating a given part of a base. In addition, it is required to eliminate completely said protective residues after immobilizing a nucleic acid on the support.

In the elimination of those protective residues, several times of change of reaction solvent and the recovery and the purification of reaction product are required, and besides the reaction sometimes requires such a long time as several hours to several days. That is, the elimination requires very tedious and time-consuming operations. In case of the method described in the official gazette of Japanese Patent Application Kokai No. 24601/1986, it is required to ligate a nucleic acid in a given direction in order to increase the effective amount of immobilized nucleotide or oligonucleotide and thus remarkably tedious operations are required as same as in the case of the method described in the official gazette of Japanese Patent Application Kokai No. 130305/1986. In addition, it is considered that an expensive enzyme is required extremely excessively, and thus this method is economically disadvantageous.

Consequently, the above two methods ae impractical for the daily use.

The object of the present invention lies in providing a hybridization carrier on which a nucleic acid can be immobilized simply and efficiently and which not only hardly causes nonspecific trap and the like of a nucleic acid but also enable the highly accurate detection, isolation and purification of a nucleic acid, and a method for preparing the same.

As a means of solving the foregoing problems of the prior art, the present invention purposes to provide a hybridization carrier which is characterized in that a single stranded polynucleotide represented by the following formula

${\text{5′-(dN)}}_{\text{n}} {\text{(dT)}}_{\text{m}} \text{-3′}$

wherein N represents adenine, guanine or cytosine, T represents thymine, n is an integer of 2 or larger, and m is an integer of 5 or larger,
is immobilized, by means of the peptide bond, on a non-porous surface of an organic polymer particle having 0.05 - 5 µm in diameter.

As a material of the organic polymer particle to be used as a carrier in the present invention (hereinafter referred to merely as "polymer particle"), water-insoluble organic polymer substance obtained by polymerizing one or more vinyl monomers composed of aromatic compound, esters or amides of α,β-unsaturated carboxylic acid, α,β-unsaturated nitrile compound, halogenic vinyl compound, conjugated-diene compound and low class fattay acid vinyl ester, for example, styrene, chlorostyrene, chloromethylstyrene, α-methylstyrene, divinylbenzene, sodium styrene sulfonate, methacrylic acid, methyl methacrylate, ethyl methacrylate, n-butyl methacrylate, 2-hydroxylethyl methacrylate, polyoxyethylene methacrylate, glycidyl methacrylate, ethylene glycol-dimethacrylic acid ester, tribromophenyl methacrylate, methacrylnitrile, methacrolein, methacrylamide, methylenebis methacrylamide, butadiene, isoprene, vinyl acetate, vinyl pyridine, N-vinyl pyrrolidone, vinyl chloride, vinyl bromide, etc.; water-insoluble and unswelling organic polymer substances obtained by chemically denaturing said organic polymer substances are enumerated.

These polymer particles can be manufactured according to well-known methods such as emulsion polymerization, suspension polymerization, solution precipitation polymerization, etc. Said polymer particles can be also obtained by dispersing an organic polymer solution in a nonsolvent to carry out the crosslinking or by dispersing a solvent. However, the manufacturing method of the polymer particle is nowise restricted to these methods. Incidentally, this polymer particle can contain stuffing, e.g., magnetic powder or the like according to demand.

The surface of polymer particle to be used in the present invention must be non-porous. Here, "non-porousness" of the surface of a polymer particle means that the particle has no hole whose long diameter is 100A or more on its surface. If the surface of polymer particle is not non-porous, that is, if there exist holes whose long diameter is 100A or more on the surface of the particle, there are cases where the sensitivity and the accuracy of the hybridization is lowered owing to such causes as a labeled probe to be used in the nucleic acid detection method and the like to be described later is trapped inside the particle and the like. Furthermore, in the isolation or the purification of nucleic acid to be described later, a substance other than the target of the isolation or the purification is trapped inside the polymer particle to cause the lowering of the isolation efficiency or the purification degree. As described above, the surface of a polymer particle is required to be non-porous, but there may exist closed cell and the like inside the particle.

The particle size of a polymer particle to be used in the present invention is 0.05 to 5 µm, preferably 0.2 to 1 µm in diameter. When the particle size is below 0.05 µm, it takes a long time for the centrifugation of particles, for example, from a sample solution after the hybridization. When the particle size is over 5 µm, it is much feared that particles may sediment in a sample solution before the hybridization undergoes sufficiently.

Furthermore, since a particle to be used in the present invention is used in an aqueous solution, the particle is required to be water-insoluble and unswelling.

A single-stranded polynucleotide carried by the carrier according to the present invention is a single-stranded deoxyribonucleic acid represented by the formula

${\text{5′-(dN)}}_{\text{n}} {\text{(dT)}}_{\text{m}} \text{-3′}$

as described above. This polynucleotide can be obtained either by chemical synthesis as described later or by artificial modifications such as the addition of one or more nucleotides to a natural nucleic acid.

This single-stranded polynucleotide is bonded to a polymer particle by means of the peptide bond, at the site of nucleotide sequence of said single-stranded polynucleotide consisting of 2 or more, generally 2 to 15 nucleotides containing primary amino residues. When the number of nucleotide containing primary amino residues is below 2, the immobilization ratio of a single-stranded polynucleotide on a polymer particle is undesirably low.

As a nucleotide containing primary amino residues, for example, deoxyadenylic acid (dA), deoxyctidylic acid (dC), deoxyguanylic acid (dG) can be enumerated. Among these, dA and dC is preferable in respect of the reactivity in the condensation with a carboxyl residue on the surface of a polymer particle. The amino residue-containing nucleotide sequence may be composed either of 1 kind of nucleotide or of 2 or more nucleotides. However, in order to avoid a base sequence accidentally becoming complemetary to a base sequence other than a specific base sequence in a nucleic acid to be hybridized with, it is preferred that the amino residue-containing nucleotide sequence is composed of 1 kind of nucleotide, particularly of dA or dC.

In case that a single-stranded polynucleotide to be used in the preparation of the present carrier contains no amino residue-containing nucleotide sequence, it is required that the amino residue-containing nucleotide sequence is introduced into its molecule prior to being subjected to the preparation method described below.

In this case, if a single-stranded polynucleotide is produced according to the chemical synthesis using, for example, a DNA synthesizer, it can be produced by so preparing a program tht the desired amino residue-containing nucleotide sequence may be formed at the desired position in its molecule. The amino residue-containing nucleotide sequence can be also introduced to the terminal of polynucleotide according to the addition reaction using terminal deoxytransferase. Furthermore, the amino residue-containing nucleotide sequence can be introduced by ligating a polynucleotide with an oligonucleotide consisting of amino residue-containing nucleotide sequences by using T4DNA ligase. In addition to those described above, the tailing reaction of nucleic acid and the ligation between nucleic acids as are well-known can be utilized (for example, refer to "Molecular Cloning", Cold Spring Harbor Laboratories, 1982).

A single-stranded polynucleotide carried by the carrier according to the present invention can hybridize with other nucleic acids which will serve for a target of the detection, the purification, the isolation, etc. The carrier according to the present invention is preferable for the hybridization, since a base sequence complementary to a poly (A) sequence portion of a nucleic acid to be hybridized with exists in a portion of a free site of said nucleotide.

Particularly, a linear single-stranded polynucleotide is immobilized on a polymer particle on the side of 5′-terminal and a base sequence complementary to a specific base sequence exists on the side of free 3′-terminal, which enables the elongation reaction of a nucleic acid by a nucleic acid-synthesizing enzyme in which said immobilized single-stranded polynucleotide acts as a primer, thus the linear single-stranded polynucelotide can be applied to the synthesis of cloned DNA or the detection of specific base sequence based on the incorporation of labeled nucleotide on a polymer particle.

A carrier according to the present invention can be prepared by reacting, for example, a polymer particle whose surface is non-porous and has a carboxyl residue and whose particle size of 0.05 to 5 µm in diameter, with the single-stranded polynucleotide described above, in the presence of a condensing agent.

The polymer particle to be used as a starting material in this method is required to have carboxyl residues which can participate in the formation of peptide bond on its surface, the other conditions are as aforementioned. Thus, in case that a polymer particle to be used as a material has no carboxyl residue on its surface, it is required to introduce a carboxyl residue on its surface previously. The number of carboxyl residues to be exist on the surface of the polymer particle is preferably at least 1, more preferably 3 or more and much more preferably 5 or more per 1nm² of surface area.

As a polymer particle having a carboxyl residue on its surface as described above and suitable for the foregoing method, for example, those commercially available under such trade names as Immutex SSM-60, SSM-58, SSM-57, G0303, G0302, G0301, G0201, G0202, G0501, L0101 and L0102; and Immutex DRB-F1, DRB-F2, DRB-F3; etc. (all are the trade names of the goods manufactured by Japan Synthetic Rubber Co., Ltd. and are carboxylated polystyrenes) can be enumerated. Furthermore, in order to introduce a carboxyl residue onto the surface of a polymer particle not carrying the same, various well-known methods are available.

As a condensing agent to be used in the foregoing preparation method, for example, water-soluble condensing agent such as carbodiimides such as 1-ethyl-3-(N,N′-dimethylamino)propylcarbodiimide or the like, Woodward's reagent K, N-ethoxycarbonyl-2-ethoxy-1,2-dihydro-quinoline (EEDQ), etc. can be enumerated as preferable ones. However, an oil-soluble condensing agent can be also used. These condensing agents are used generally in the amount of 1 to 20 moles, preferably in the amount of 2 to 10 moles per 1 equivalent of carboxyl residue possessed by a polymer particle to be used on its surface. This reaction can be carried out, for example, by putting a polymer particle having a carboxyl residue on its surface and a single-stranded polynucleotide containing an amino residue-containing nucleotide sequence into a reactor of suitable volume and then adding the foregoing condensing agent thereto. The amount of polymer particle to be used is generally 0.5 to 500 g, preferably 5 to 50 g per 1 mmol of single-stranded polynucleotide containing amino residue-containing nucleotide sequence. It is generally sufficient that the reaction is carried out in an aqueous medium of pH on the order of 3 to 11 at 4 to 70°C for 5 minutes to one night.

The present carrier can be obtained by condensing primary amino residues of the amino residue-containing nucleotide sequences possessed by a single-stranded polynucleotide with a carboxyl residue on the surface of a polymer particle according to the method described above to form a peptide bond.

The present carrier and the preparation method described above can be applied to the detection of nucleic acid according to the sandwich method and the competitive method.

According to the sandwich method, two different and nonoverlapping specific base sequences, one of which is a poly (A) sequence are firstly selected from the base sequences contained in a nucleic acid to be detected from a specimen. The carrier of the present invention carries a polynucleotide containing a sequence complementary to poly (A) sequence. Then a labeled nucleic cid probe is prepared by labeling a nucleic acid complementary to the above specific base sequence other than poly (A) sequence, with an appropriate label. When these carrier and labeled nucleic acid probe are subjected to the hybridization with a nucleic acid in a specimen, the nucleic acid of the carrier and the labeled nucleic acid probe are bonded in the form of sandwhiching said nucleic acid in the specimen according to the hybridization if there exists a nucleic acid containing a poly (A) sequence to be detected in the specimen. Then, the labeled nucleic acid bonded to the carrier is detected as a tracer to make the detection of a nucleic acid containing a specific base sequence possible.

According to the competitive method, a single-stranded nucleic acid containing a base sequence complementary to a poly (A) sequence of a mRNA to be detected in the specimen is firstly bonded to a polymer particle to prepare a carrier of the present invention. On the other hand, a nucleic acid containing a poly (A) sequence substantially equal to that of a nucleic acid to be detected in the specimen is labeled to prepare a probe. Then, the foregoing carrier, the specimen and the probe are mixed and subjected to the hybridization reaction, where, if there exists a nucleic acid containing a specific base sequence to be detected in the specimen, said nucleic acid competes with the probe for the hybridization with the single-stranded nucleic acid bonded to the carrier. In case that there exists no nucleic acid to be detected in the specimen, the probe alone hybridized with the single-stranded nucleic acid on the carrier. The amount of probe to be hybridized with a single-stranded nucleic acid on a carrier decreases in direct proportion to the amount of nucleic acid to be detected in the specimen. Thus, a nucleic acid containing a specific base sequence can be specifically determined by detecting said probe.

In the sandwich method and the competitive method described above, a specimen containing nucleic acids, labeled nucleic acid probes and a buffer suitable for the hybrid formation are added to nucleic acid-bonded polymer particles, followed by allowing to stand for approx. 2 hours at an appropriate (e.g. 37°C) temperature, thereby forming hybrids. Then, the excess labeled nucleic acid probes are removed by the centrifugation or the like, followed by analytically detecting the labels bonded to the carriers.

A probe to be used in the detection of nucleic acid described above is the one consisting of a single-stranded nucelic acid as a direct target of the detection and being labeled with an easily detectable label. A label to be used is a substance which can be easisly detected as a tracer such as enzymologically active residue, fluor, chromophor, luminophor, specifically bondable ligand, heavy metal and radioactive isotope, by which a labeled probe trapped on a carrier owing to the hybridization can be easily detected.

The abovementioned sandwich method can be applied to the detection of pathogens such as bacterium, virus, etc., the screening of antibiotics and antiviral agents, the diagnosis of hereditary disorder and the detection of cancer cell.

The present carrier can be applied to the isolation of mRNA containing poly (A) sequence.

Said isolation of nucleic acid is carried out by firstly immobilizing a single-stranded nucleic acid containing a base sequence complementary to a poly (A) sequence in a mRNA sample on a polymer particle to form the present carrier, then making said carrier hybridize with a mRNA containing a poly (A) sequence in the foregoing mRNA sample and then isolating a mRNA hybridized with the foregoing carrier.

In this method, when isolating a nucleic acid to be isolated as a hybrid with the foregoing carrier from nucleic acids not hybridized with the nucleic acids on the carrier and other impurities, said nucleic acid can be isolated by firstly removing these unnecessary nucleic acids and the like by the centrifugation or the like, then subjecting the resulting products to a heat treatment or to a treatment with alkali, formamide or urea, such as to cut a hydrogen bond between nucleic acids constituting a hybrid and then recovering a nucleic acid containing the desired specific base sequence.

Said isolation of nucleic acid is useful in isolating, mRNA encoding the synthesis of protein useful for human being, animal, plant or microorganism from samples such as crude extract of cell and the like.

Incidentally, the conditions such as a buffer, temperature, reaction time, etc. suitable for the hybridization conducted in the detection and the isolation of nucleic acid described above are obvious to those skilled in the art.

Furthermore, the present carrier can be applied to the cloning of the isolated nucleic cid, particularly of RNA on said carrier as aforementioned.

That is, a single-stranded nucleic cid containing a sequence complementary to a part of sequence as a target of the cloning in its 3′-portion (hereinafter referred to as "immobilized nucleic acid") on a particle to form the present carrier, then said carrier is hybridized with a nucleic acid comprised of sequences as targets of the cloning in a nucleic acid sample (hereinafter refered to as "target nucleic acid") and then a reverse transcriptase is made to act while making said target nucleic acid and said immobilized nucleic acid serve respectively as a template and a primer, whereby a DNA containing a sequence complementary to the target nucleic acid (hereinafter referred to merely as "primary strand of cloned DNA") can be synthesized.

Furthermore, by applying Okayama-Berg method (H. Okayama and P. Berg; Molecular and Cellular Biology, 2, 161-170, 1982), Gubler-Hoffmann Method (U. Gubler and G.J. Hoffmann; Gene, 25, 263-269, 1983), the cloning of cDNA by the use of S₁ nuclease (Williams, J.G.; "Genetic Engineering", vol. 1, p. 1-59, Academic Press, London and New York, 1981), etc., a sequence complementary to the primary strand of a cloned DNA, that is a DNA containing a sequence equal to the target nucleic cid (hereinafter referred to merely as "secondary strand of cloned DNA) is synthesized, whereby the target nucleic cid can be cloned.

Besides, in the above method, by inserting a recognition sequence for a restriction enzyme between a sequence consisting of 2 or more nucleotides containing primary amino residues in the immobilized nucleic acid and a sequence complementary to a part of the target nucleic acid in 3′-portion also in the immobilized nucleic cid, the cloned DNA can be scissored out from the carrier with the restriction enzyme after synthesizing the secondary stand of the cloned DNA, whereby the cloned DNA can be recovered.

Furthermore, after synthesizing the primary strand of a cloned DNA by the use of an immobilized nucleic acid containing a recognition sequence for the restriction enzyme, oligo(dA), oligo(dT), oligo(dC) or oligo(dG) is added to the 5′-terminal of the cloned DNA according to the addition reaction, then the target nucleic cid is eliminated by the decomposition or the denaturation to form a hybrid between a single-stranded nucleic acid containing not only a sequence complementary to the added oligonucleotide (addition sequence) in its 3′-portion but also a recognition sequence for the restriction enzyme and the tailed site of the primary strand of a cloned DNA. After synthesizing the secondary strand of a cloned DNA by making a nucleic acid elongation enzyme represented by DNA polymerase act while making said single-stranded nucleic acid complementary to the addition sequence and the primary strand of a cloned DNA serve respectively as a primer and a template, a restriction enzyme is made to act to scissor out a cloned DNA having a recognition site for a restriction enzyme at its both terminal (5′- and 3′-terminal) from the carrier to make the recovery of the cloned DNA possible. The obtained cloned DNA can be incorporated into a plasmid which similarly having a recognition site for the restriction enzyme as it is. In this method for synthesizin a cloned DNA, the single-stranded nucleic acid complementary to the tailed nucleic cid can be the present hybridization carrier immobilized on a polymer particle.

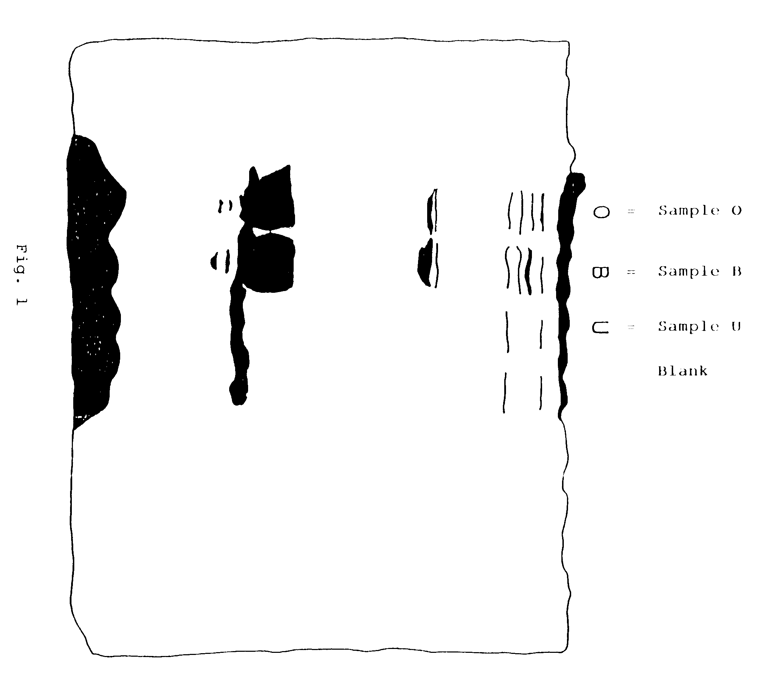

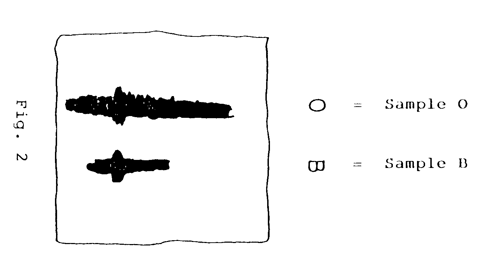

Figs. 1 and 2 are autoradiograms obtained after subjecting the reaction solutions of globin protein synthesized in Example 4 to the electrophoresis and then drying.

Hereinafter, the present invention will be described specifically, referring to examples. However, the present invention is nowise restricted to the examples.

### Example 1

### (1) Preparation of polynucleotide 5′-(dC)ₙD(dT)₃₀-3′ (n=0, 1, 3, 7 or 10)

5 kinds of polynucleotides whose nucleotide sequences are represented by 5′-(dC)ₙ(dT)₃₀-3′ (n=0, 1, 3, 7 or 10), [wherein 5′- on the left end and 3′- on the right end respectively stand for 5′-terminal and 3′-terminal (which will stand for the same meanings hereinafter)] were synthesized by using a DNA synthesizer (Model 381A manufactured by Applied Bio System, Inc.) according to the solid phase method, that is, β-cyanoethyl method. The scission and the collection of the synthesized polynucleotide from the solid phase were carried out according to the manual for said synthesizer.

Then, each of the obtained polynucleotides was dissolved in 250 µl each of TNE buffers (a buffer consisting of 10mM Tris-HCl buffer of pH 7.5, 100mM sodium chloride and 1mM ethylenediaminetetraacetic acid), followed by subjecting the gel filtration using a Sephadex G-50 column (manufactured by Pharmacia Fine Chemicals, Inc.) swollen with the TNE buffer. The elutes by the gel filtration were fractioned into 0.5-ml portions and collected successively. Then, the absorbance of each fraction at 260nm was measured, thereby discriminating untrapped fractions to collect the same. From the collected polynucleotide solutions, were purified polynucleotides by the precipitation with ethanol. Then, each purified polynucleotide was dissolved in 500 µl each of sterile water, followed by measuring the absorbance at 260nm to determine each polynucleotide.

As a result of the above procedures, approx. 1mg each of 5 kinds of polynucleotides as aforementioned was obtained in the form of 500 µl ech of aqueous solution.

### (2) Labeling with ³²P

Each of 5 kinds of polynucleotide obtained in (1) was labeled with ³²P as follows.

1 µl of polynucleotide solution obtained in (1) (containing 2 µg of polynucleotide), 1 µl (8U in terms of enzymatic activity) of polynucleotide kinase (manufactured by Boehringer Mannheim, Inc.), 5 µl (50µCi in terms of radiation dose) of γ-³²P-deoxyadenosine triphostate, 5 µl of 10-fold concentrated 5′-terminal phosphorylation buffer and 38 µl of sterile water were poured into a 1.5-ml microcentrifuge tube, followed by mixing to incubate at 37°C for 30 minutes.

In the next place, after removing polynucleotide kinase from the reaction solution according to the phenol extraction method (refer to "Molecular Cloning", Cold Spring Harbor Laboratories, p. 458, 1982), the thus treated reaction solution was subjected to the gel filtration using a 1-ml Sephadex G-50 column (manufactured by Pharmacia Fine Chemicals, Inc.) swollen with TNE buffer.

Incidentally, the 10-fold concentrated 5′-terminal phosphorylation buffer used above had the following composition.
- Composition:: 0.5M Tris-HCl buffer (pH 7.6)
0.1M magnesium chloride
50mM dithiothreitol
1mM spermidine
1mM ethylenediaminetetraacetic acid
The elute by the gel filtration was fractioned into 100-µl portions and collected. Then, the radiation dose of each fraction was measured according to Cerenkov's counting method (c.f. H. Braunsberg et al. Anal. Biochem. 10, 86, 1965), followed by discriminating and collecting the untrapped fractions. As a result of it, 200 µl each of TNE buffers containing any of 5 kinds of polynucleotides of 5′-(dC)ₙ(dT)₃₀-3′ (wherein n=0, 1, 3, 7 or 10) labeled with ³²P was obtained. These were solutions each having a radiation dose of 400,000 cpm/µl.

### (3) Immobilization on polymer particle having carboxylated surface

500 µl of 0.0001N HCl suspension containing 1(w/v)% Immutex SSM-60 (manufactured by Japan Synthetic Rubber, Co., Ltd.) which is a polymer particle of 0.36 µm diameter whose surface is carboxylated to have 9 carboxyl residues per 1nm² of surface area, 1nmol of polynucleotide having a nucleotide sequence of 5′-(dC)ₙ(dT)₃₀-3′ (wherein n=0, 1, 3, 7 or 10) as was synthesized in (1), 500 µl of 0.0001N HCl solution containing 0.5(w/v)% 1-ethyl-3-(N,N′-dimethylamino)propylcarbodiimide and 400,000cpm equivalent of ³²P-labeled polynucleotide (having the same dC number as non-labeled polynucleotide) were poured into a 1.5-ml microcentrifuge tube and mixed overnight in a thermostat whose temperature was set to 50°C, whereby the ³²P-labeled polynucleotide was bonded to a polymer particle by the peptide bond. Then, the resulting product was centrifuged at 10,000rpm for 5 minutes to recover a hybridization carrier as a precipitate. The hybridization carrier was washed by repeating the operations of adding thereto 1 ml of binding buffer (a buffer consisting of 10mM Tris-HCl buffer of pH 7.5, 500mM sodium chloride, 0.1(w/v)% sodium dodecylsulfate and 1mM ethylenediaminetetraacetic acid) to redisperse the same, then centrifuging the dispersion at 15,000rpm for 5 minutes and then recovering thus centrifuged hybridization carrier. The remaining radioactivity on the thus obtained hybridization carrier was measured according to Cerenkov's counting method. The immobilization ratio was obtained by dividing said remaining radioactivity value by the count number of the added ³²P-labeled oligonucleotide (400,000cpm). The results are given in Table 1.

**Table 1**

| Number of n in (dC)ₙ(dT)₃₀ | Immobilization Ratio (%) |
|---|---|
| 0 | 9 |
| 1 | 52 |
| 3 | 72 |
| 7 | 89 |
| 10 | 93 |

### Example 2

### Determination of polyadenylic acid sequence contained in mRNA of vaccinia virus

### (1) Preparation of hybridization carrier

Polynucleotide of 5′-(dC)₁₀(dT)₃₀-3′ having a base sequence of 5′-CCCCCCCCCCTTTTTTTTTTTTTTTTTTTTTTTTTTTTTT-3′ was firstly synthesized in a DNA synthesizer. The (dT)₃₀ sequence on the side of 3′-terminal of this polynucleotide was complementary to the poladenylic acid (hereinafter abbreviated as "poly(A)") sequence contained in a mRNA of vaccinia virus. The (dC)₁₀ sequence on the side of 5′-terminal is a site to be used for the immobilization onto a polymer particle.

2.5 ml of 0.0001N HCl suspension containing 1(w/v)% surface-carboxylated polymer particle (Immutex SSM-60 manufactured by Japan Synthetic Rubber Co., Ltd.), 2.5 ml of 0.0001N HCl solution containing 5 mg/ml of 1-ethyl-3-(N,N′-dimethylamino)propylcarbodiimide, 100 µg of the above synthetic polynucleotide and 10,000cpm of the above synthetic polynucleotide labeled with ³²P in the same manner as in Example 1-(2) were mixed and reacted at 50°C for 6 hours. After the reaction, a suspension of hybridization carrier was centrifuged at 3000rpm for 15 minutes to recover the hybridization carrier. The recovered hybridization carrier was washed by repeating the operations of suspension in 10 ml of 0.0001N HCl and centrifugation. After the thoroughly washing, the hybridization carrier was suspended in a solution consisting of 10mM tris-HCl buffer (pH 7.5), 0.1M sodium chloride, 1mM ethylenediaminetetraacetic acid (pH 7.5) and 0.1(w/v)% sodium dodecylsulfate to give a total volume of 5 ml. Thus obtained 0.5(w/v)% suspension of hybridization carrier was preserved at 4°C.

It was found out that the immobilization ratio of polynucleotide on thus prepared hybridization carrier was 98%, that is, 98 µg of polynucleotide was immobilized on the polymer particle on the side of 5′-terminal.

### (2) Preparation of poly(A)-containing mRNA of vaccinia virus

Vaccinia virus was washed with a surfactant [0.5(w/v)% NP40] and centrifuged to recover the same as a pellet. To this pellet, were added 2000 µl of solution containing 50mM Tris-HCl buffer (pH 8.6), 7.5mM magnesium chloride, 20mM β-mercaptoethanol, 5mM adenosine triphosphate, 5mM cytidine triphosphate, 5mM guanosine triphosphate, 5mM uridine triphosphate, 0.5% NP40, 0.3mM S-adenosylmethionine and RNase inhibitor, followed by incubating at 37°C for 3 hours. Then, a supernatant obtained by centrifuging the reaction solution at 15,000rpm for 10 minutes was extracted with phenol, followed by subjecting to the column chromatography using Sephadex G-100 (manufactured by Pharmacia Fine Chemicals, Inc.), thereby collecting untrapped fractions. The collected fractions were applied to a column packed with oligo(dT)-cellulose to fraction the same into mRNAs containing poly(A) sequences and those not containing poly(A) sequences, whereby 18 µg of mRNA containing poly(A) sequence and 10 µg of mRNA not containing poly(A) sequence were obtained.

### (3) Determination of mRNA containing poly(A) sequence

### (3-1)

20 µl of suspension of hybridization carrier prepared in (1) was poured in a microcentrifuge tube, followed by adding 1 ml of 100mM Tris-HCl buffer (pH 8.3) thereto. Then, the thus treated suspension was centrifuged at 10,000rpm for 5 minutes to recover the hybridization carrier.

To this hybridization carrier, were added 49 µl of solution with the following composition:
100mM Tris-HCl buffer (pH 8.3)
160mM potassium chloride
20mM β-mercaptoethanol
1mM deoxyadenosine triphosphate
1mM deoxyguanosine triphosphate
1mM deoxythymidine triphosphate
0.1mM deoxycytidine triphosphate
10µCi α-³²P-deoxycytidine triphosphate
10mM magnesium chloride
8U RNase inhibitor.

Furthermore, 10ng, 1ng, 0.1ng or 0ng of such mRNA containing poly(A) sequence prepared in (2) as was previously subjected to the heat treatment at 65°C for 5 minutes and to the rapid cooling on ice was added the above solution, followed by incubating at 43°C for 10 minutes to form a hybrid. Then, 1 µl (corresponding to 17U) of reverse transcriptase (Life Science, Inc.) was added to the reaction solution, followed by incubating at 43°C for 15 minutes. Thereafter, 1 µl of 0.5M ethylenediaminetetraacetic cid (pH 8.0) was added to the incubated reaction solution, followed by washing with a solution consisting of 10mM Tris-HCl buffer (pH 8.0), 100mM sodium chloride and 1mM ethylenediaminetetraacetic acid (pH 8.0) while filtration with suction using a 0.22-µm membrane filter. After the washing, the membrane filter was dried and the radioactivity of the obtained hybridization carrier filtered on the membrane filter was measured in a 5-ml toluene-based liquid scintillator (Beckman).

### (3-2)

The experiment was carried out by following the same procedure as described in the foregoing (3-1) except that mRNA not containing poly(A) sequence prepared in (2) was used instead of that containing poly(A) sequence. The radioactivity of the obtained hybridization carrier filtered on the membrane filter was measured.

### (3-3)

The results of the determination in (3-1) and (3-2) are given in Table 2.

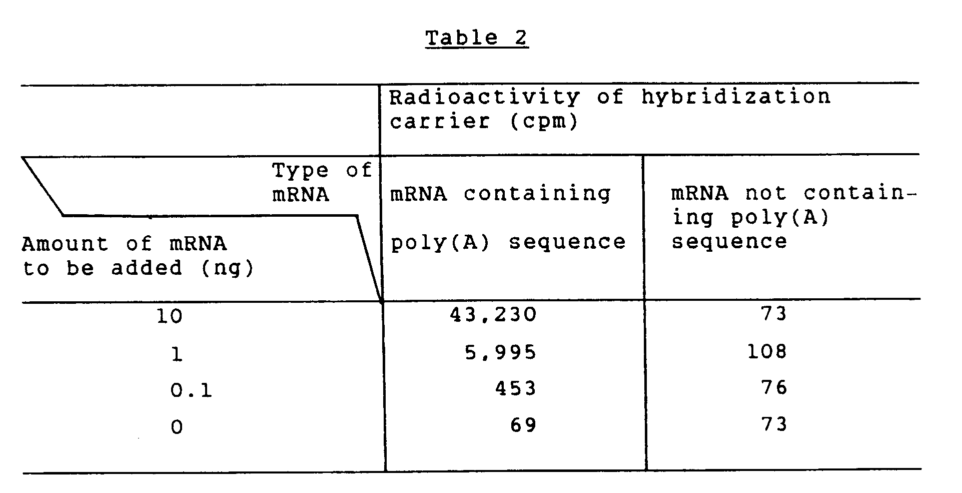

### Example 3

### Isolation and purification of Poly(A)-containing mRNA of Vaccinia virus

### (1) Preparation of poly(A) containing mRNA of vaccinia virus

The preparation was carried out in the same manner as in Example 2-(2) except that the concentration of uridine triphosphate contained in a solution to be added to the pellet obtained by the centrifugation after treating vaccinia virus with NP40 was changed to 0.5mM and that 50Ci of α-³²P-uridine triphosphate was added to said solution, thereby obtaining poly(A) sequence-containing mRNA of vaccinia virus.

### (2) Isolation and purification of mRNA from impurities

10 µl [0.5(w/v)%] of suspension of hybridization carrier prepared in the same manner as in Example 2-(1) was poured into a microcentrifuge tube, followed by adding 50 µl of 10mM Tris-HCl buffer (pH 7.5) containing 0.5M sodium chloride, 0.1(w/v)% sodium dodecylsulfate and 1mM ethylenediaminetetraacetic acid, 50 µg of bovine serum albumin, 30 µg of ribosomal RNA, 30 µg of transfer RNA and 300ng of poly(A) sequence-containing mRNA prepared in the above (1) and subsequently incubating at 37°C for 10 minutes. Thereafter, the incubated product was centrifuged at 10,000rpm for 5 minutes to separate the same into a supernatant and a solid, followed by measuring each radioactivity according to Cerenkov's counting method. The hybridization efficiency was calculated at 94.7% according to the following equation:

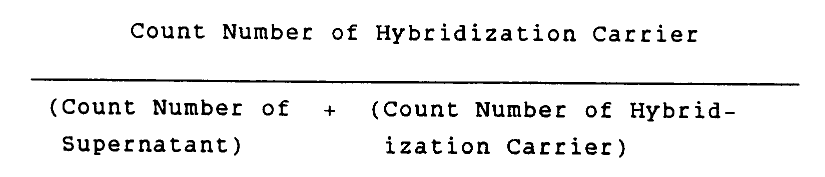

Then, 10 µl of 10mM Tris-HCl buffer (pH 7.5) containing 0.1(w/v)% sodium dodecylsulfte and 1mM ethylenediaminetetraacetic acid was added to the hybridization carrier prepared in the above (1), followed by heating at 65°C for 5 minutes. The resulting product was then centrifuged at 10,000rpm for 5 minutes to separate the same into the supernatant and the hybridization carrier, followed by measuring each radioactivity according to Cerenkov's counting method. The recovery was calculated at 91.6% according to the following equation:

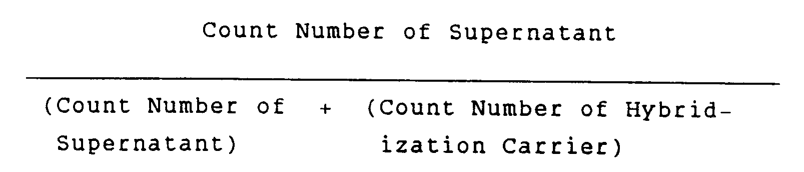

### Example 4

### Isolation and Purification of Globin mRNA

### (1) Isolation and purification of globin mRNA

200 µl of 0.5(w/v)% suspension of hybridization carrier prepared in the same manner as in Example 2-(1) was poured into a 1.5-ml microcentrifuge tube and then centrifuged at 15,000rpm for 5 minutes, followed by discarding the supernatant.

In the next place, 100 µl of binding buffer having the same composition as that in Example 1-(3) and 20 µl of sterile aqueous solution containing 50 µg/ml of globin mRNA (hereinafter referred to as "Sample 0") (manufactured by Bethesda Research, Inc.) were added to this centrifuge tube, followed by mixing the hybridization carrier thoroughly. The mixture was then incubated at 37°C for 10 minutes. Thereafter, the incubated mixture was centrifuged at 15,000rpm for 5 minutes to collect supernatant (hereinafter referred to as "Sample U"). To the precipitated hybridization carrier, was added 100 µl of elution buffer [a buffer consisting of 10mM Tris-HCl buffer (pH 7.5), 0.1(w/v)% sodium dodecylsulfate and 1mM ethylenediaminetetraacetic acid) to redisperse the hybridization carrier therein. The redispersed hybridization carrier was heated at 65°C for 5 minutes, followed by centrifuging at 4°C at 15,000rpm for 5 minutes to collect supernatant yielded (hereinafter referred to as "Sample B").

In the next place, Samples U and B were respectively subjected to the ethanol precipitation to recover nucleic acids contained in each sample as precipitates.

### (2) Synthesis of globin

In order to confirm that the globin mRNA obtained in the above (1) has a complete structure, Sample U, B or O was added to a translation kit (manufactured by du Pont) to make globin mRNA in each sample synthesize globin protein. Regarding reagents required for this synthesis, those provided with said kit were used. The synthesis was carried out according to the manual for said kit.

After the completion of synthesis reaction of globin protein, each reaction solution was subjected to the electrophoresis using 15(w/v)% SDS-PAGE (polyacrylamide gel). Then, the gel was dried and subjected to the autoradiography. For the comparison, the blank not containing mRNA was also subjected to the autoradiography. A sketch of the obtained autoradiograms is given in Fig. 1.

From the above autoradiograms, it could be confirmed that the globin mRNA isolated in Sample B did not lose the function of mRNA of Sample 0 and tht no globin mRNA remained in Sample U.

### (3) Synthesis of complementary DNA (cDNA) of globin

Samples B and O described in the above (1) were added to cDNA synthesis kits (manufactured by Amersham Int., Ltd.) to synthesize complementary DNAs.

This synthesis was carried out using reagents provided with the kit according to the manual for said kit, except that 50 µCi of α-³²P-deoxycytidine triphosphate were used. After the completion of synthesis reaction of a cDNA of globin, 5 µl of reaction solution was subjected to the electrophoresis using 1(w/v)% agarose gel. Then, the gel was dried and subsequently subjected to the autoradiography. A sketch of the obtained autoradiograms is given in Fig. 2.

From these autoradiograms, it was found out that the purity of the globin mRNA of Sample B is higher than that of Sample O.

A hybridization carrier according to the present invention is useful for the detection, the isolation, the purification, etc. of nucleic acid containing specific base sequence, and hardly causes the nonspecific trap of nucleic acid and other trouble in the hybridization to enable the highly accurate detection, isolation, purification, etc.

In addition, the present hybridization carrier on which a nucleic acid is immobilized in a state of 3′-terminal of the nucleic acid being free is useful for the cDNA synthesis and the like. The preparation method to be provided by the present invention is a very practical method in that a nucleic acid can be simply and efficiently immobilized on such a carrier as above.

## Claims

1. A hybridization carrier which is characterized in that a single stranded polynucleotide represented by the following formula
${\text{5′-(dN)}}_{\text{n}} {\text{(dT)}}_{\text{m}} \text{-3′}$
wherein N represents adenine, guanine or cytosine, T represents thymine, n is an integer of 2 or larger, and m is an integer of 5 or larger,
is immobilized by means of the peptide bond, on a non-porous surface of an organic polymer particle having 0.05 - 5 µm in diameter.

2. A process for preparing a hybridization carrier which comprises reacting an organic polymer particle whose surface is non-porous and carries a carboxyl residue and whose particle size in diameter is 0.05 to 5 µm with a single-stranded polynucleotide represented by the following formula
${\text{5′-(dN)}}_{\text{n}} {\text{(dT)}}_{\text{m}} \text{-3′}$
wherein N represents adenine, guanine or cytosine, T represents thymine, n is an integer of 2 or larger, and m is an integer of 5 or larger,
in the presence of a condensing agent.

## Patentansprüche

1. Hybridisierungsträger, der gekennzeichnet ist dadurch, daß ein einzelsträngiges Polynukleotid mit der folgenden Formel:
${\text{5'-(dN)}}_{\text{n}} {\text{(dT)}}_{\text{m}} \text{-3'}$
worin N Adenin, Guanin oder Cytosin bedeutet, T Thymin bedeutet, n eine ganze Zahl von 2 oder mehr ist und m eine ganze Zahl von 5 oder mehr ist, immobilisiert wird über die Peptidbindung auf einer porenfreien Oberfläche eines organischen Polymerteilchens mit einem Durchmesser von 0,05 bis 5 µm.

2. Verfahren zur Herstellung eines Hybridisierungsträgers, das umfaßt, daß man ein organisches Polymerteilchen, dessen Oberfläche porenfrei ist und Carboxylreste trägt und dessen Teilchengröße 0,05 bis 5 µm im Durchmesser ist, mit einem einzelsträngigen Polynukleotid der Formel
${\text{5'-(dN)}}_{\text{n}} {\text{(dT)}}_{\text{m}} \text{-3'}$
worin N Adenin, Guanin oder Cytosin bedeutet, T Thymin bedeutet, n eine ganze Zahl von 2 oder mehr ist und m eine ganze Zahl von 5 oder mehr ist, in Gegenwart eines Kondensationsmittels umsetzt.

## Revendications

1. Support d'hybridation caractérisé par le fait qu'on immobilise un polynucléotide monobrin représenté par la formule suivante
${\text{5' - (dN)}}_{\text{n}} {\text{(dT)}}_{\text{m}} \text{- 3'}$
dans laquelle N représente l' adénine, la guanine ou la cytosine, T représente la thymine, n est un nombre entier égal ou supérieur à 2, et m est un nombre entier égal ou supérieur à 5,
au moyen de la liaison peptidique, sur une surface non-poreuse d'une particule de polymère organique de diamètre compris entre 0,05 et 5 µm.

2. Procédé pour la préparation d'un support d'hybridation comprenant le fait qu'on fait réagir une particule de polymère organique dont la surface est non-poreuse et porte un radical carboxyle et dont la taille de particule est comprise entre 0,05 et 5 µm en diamètre avec un polynucléotide monobrin représenté par la formule suivante
${\text{5' - (dN)}}_{\text{n}} {\text{(dT)}}_{\text{m}} \text{-3'}$
dans laquelle N représente l' adénine, la guanine ou la cytosine, T représente la thymine, n est un nombre entier égal ou supérieur à 2, et m est un nombre entier égal ou supérieur à 5,
en présence d'un agent de condensation.
